# EUROPEAN PATENT SPECIFICATION

(11) **EP 0 584 723 B1**
(45) Date of publication and mention of the grant of the patent: **05.02.1997**
(21) Application number: 93113273.2
(22) Date of filing: 19.08.1993
(51) Int. Cl.: A61B 17/28

(54) **Handle for endoscopic surgical instruments and jaw structure**
Handgriff und Zungenbacken eines endoskopischen chirurgischen Instruments
Poignée et mâchoires d'instrument chirurgical endoscopique

(30) Priority: 19.08.1992 US 932230
(43) Date of publication of application: 02.03.1994
(73) Proprietor: United States Surgical Corporation, Norwalk, Connecticut 06856 (US)
(72) Inventor: Green, David T., Westport, CT 06880 (US); Aranyi, Ernest, Easton, CT 06612 (US); Tovey, Ian J., Milford, CT 06460 (US)
(74) Representative: Marsh, Roy David

(56) References cited:
- EP-A- 0 543 107
- US-A- 2 796 065
- US-A- 4 760 848
- US-A- 5 026 379
- "Jarit" catalogue of surgical instruments / endoscopic instruments, Petit point Babcock forceps and Babcock tissue forceps.

## Description

### BACKGROUND OF THE INVENTION

### 1. Field of the Invention

The present invention relates to surgical instruments, and more particularly relates to improvements in jaw structure for an endoscopic or laparoscopic surgical instrument having reciprocating jaw members which pivot in response to the opening and closing of the handle members, where movement of the handle members is translated through an elongated tubular body member to open and close the jaw mechanism. The invention is described below as part of the instrument which is the subject of Applicant's EP-A-0 543 107, to which the reader is referred for a full description of what is shown in Figures 1 to 33 of the accompanying drawings. EP-A-0543107 was published May 26, 1993, a date later than the presently declared priority dated, so can belong to the prior art only by virtue of Article 54(3) EPC.

### 2. Discussion of the Prior Art

U.S. Patent No. 1,452,373 to Gomez discloses a typical locking mechanism for a surgical instrument, in which a plurality of ribs are provided on an extension of the handle member which engage a similar rib member on the opposite handle. Once engaged, the handles must be moved away from each other perpendicular to their longitudinal axis to disengage the locking mechanism to release the jaw mechanism.

U.S. Patent No. 4,896,661 to Bogert et al. disclose a surgical instrument having a ratchet mechanism positioned on the handle members which includes a curved rack member attached to one handle member which passes through a slot in the other handle member. A releasable pawl member is provided on the second handle to engage the rack member and provide a means for releasing the ratchet.

U.S. Patent No. 4,935,027 to Yoon discloses a surgical instrument having a ratchet mechanism positioned between the handle members. A rack member is provided which extends from one handle and passes through a slot in the second handle to lock the handles in place. Pivoting the rack member away from corresponding grooves in the slot will release the ratchet mechanism.

U.S. Patent No. 4,428,374 to Auburn discloses a surgical instrument having means for positioning and holding the handle members in relation to each other. A rack member is provided on one handle member which extends through a slot in the second handle member in which a releasable pawl mechanism is provided to engage and disengage the ratcheting mechanism.

Manipulation of typical surgical instruments having jaw mechanisms creates risks to the surrounding tissue, particularly the tissue coming in contact with the distal end of the instrument where opposing jaw members meet. These risks are compounded in the endoscopic and laparoscopic environment there maneuverability of instruments as well as visibility in the entire surgical site are limited.

Furthermore, with respect to jaw members and, in particular, jaw members used for endoscopic or laparoscopic procedures where a limited number of access points to the surgical area through trocar cannulas is desired, it is necessary to keep instrumentation to a minimum. Previous devices function very well for their specific purposes, however, in conventional jaw mechanisms the gripping surface area is limited and separate instruments are required having different jaw structures for performing different functions, for example, forceps for gripping and pulling away tissue and graspers for atraumatically holding tubular vessels. Having to provide these separate instruments increases the time to perform the necessary procedures by requiring exchanging instruments in the cannulas or by creating additional access points using additional trocars.

A problem addressed by the present invention was to find jaw mechanisms which have novel multiple tissue handling portions which add gripping surface while keeping risks to surrounding tissue low and allow surgeons to use fewer instruments, therefore, reducing the time required to perform procedures. US-A 5 026 379 discloses a surgical instrument in accordance with the pre-characterising part of claim 1 below.

### SUMMARY OF THE INVENTION

The present invention provides a novel endoscopic or laparoscopic surgical device defined in claim 1 below.

### BRIEF DESCRIPTION OF THE DRAWINGS

The foregoing objects and other features of the invention will become more readily apparent and may be understood by referring to the following detailed description of an illustrative embodiment of the endoscopic or laparoscopic surgical instrument having an internal ratchet mechanism, den in conjunction with the accompanying drawings, in which:
Figure 1 illustrates a perspective view of an endoscopic or laparoscopic surgical instrument possessing the ratchet mechanism, as disclosed in Applicant's EP-A-0 543 107;
Figure 2 illustrates a side plan view and partial cut-away of the surgical instrument of Figure 1;
Figure 3 illustrates an exploded side cut-away view of the device of Figure 2 showing in detail the ratchet mechanism;
Figures 4 and 5 illustrate a side plan view and a perspective view, respectively, of the ratchet mechanism of the embodiment of Figure 1;
Figure 6 illustrates a perspective view of an alternate embodiment of the endoscopic or laparoscopic surgical instrument;
Figure 7 illustrates a side plan view of the laparoscopic surgical instrument of Figure 6;
Figure 8 illustrates a top plan view of the device of Figure 6;
Figure 9 illustrates a side cut-away view of the endoscopic or laparoscopic surgical instrument of Figure 6;
Figure 10 illustrates a further embodiment of the endoscopic or laparoscopic surgical instrument;
Figure 11 illustrates a side view of the device of Figure 10;
Figure 12 illustrates a side cut-away view of the device of Figure 10;
Figure 13 illustrates another embodiment of the endoscopic or laparoscopic surgical instrument;
Figure 14 illustrates a side plan view of the device of Figure 13;
Figure 15 illustrates a side cut-away view of the device of Figure 13;
Figure 16 illustrates a perspective view of a further embodiment of the endoscopic or laparoscopic surgical instrument;
Figure 17 illustrates a side plan view of the device of Figure 16;
Figure 18 illustrates a side cut-away view of the device of Figure 16;
Figure 19 illustrates a plan view of an embodiment of the actuation means for use with the instrument;
Figure 20 illustrates a plan view of the stop means for use with the rotation knob of the instrument;
Figure 21 illustrates a top plan view of a jaw member prior to being formed;
Figure 22 illustrates a top plan view of the jaw member of Figure 21 wherein a proximal end is bent relative to the distal end;
Figure 23 illustrates a side plan view of the jaw member of Figure 22 wherein the distal end is formed in a semicircular configuration;
Figure 24 illustrates a perspective view in partial phantom of a jaw mechanism with the jaw members closed;
Figure 25 illustrates a perspective view of the jaw mechanism of Figure 24 with the jaw members in the open position;
Figure 26 illustrates a perspective view of an endoscopic instrument incorporating the jaw mechanism of Figure 24;
Figure 27 illustrates a top plan view of a jaw member in accordance with another embodiment of the instrument;
Figure 28 illustrates a front plan view in cross section of a jaw member taken along line 28-28 of Figure 27;
Figure 29 illustrates a side plan view of the jaw member of Figure 27;
Figure 30 illustrates a perspective view in partial phantom of a jaw mechanism in accordance with jaw members of Figure 27 in the closed position;
Figure 31 illustrates a perspective view of the jaw mechanism of Figure 30 with the jaw members in the open position;
Figure 32 illustrates perspective view of a jaw member in accordance with another embodiment of the instrument;
Figure 33 illustrates a side plan view in cross section of an endoscopic instrument incorporating the jaw member of Figure 32;
Figure 34 illustrates a top view of a jaw member in accordance with one embodiment of the present invention prior to being formed;
Figure 35 illustrates a bottom view of the jaw member of Figure 34 wherein a proximal end is bent relative to the distal end;
Figure 36 illustrates a side plan view of the jaw member of Figure 35 wherein the distal end is formed having a semicircular configuration and a serrated portion extending distally therefrom;
Figure 37 illustrates a perspective view of an endoscopic instrument utilizing the jaw member of Figure 36;
Figure 38 illustrates a partial side view of the distal end of a jaw member in accordance with another embodiment of the present invention;
Figure 39 illustrates a partial side view of the distal end of a jaw member in accordance with another embodiment of the present invention; and
Figure 40 illustrates a partial side view of the distal end of opposing jaw members having abutting teeth.

### DETAILED DESCRIPTION OF THE PREFERRED EMBODIMENTS

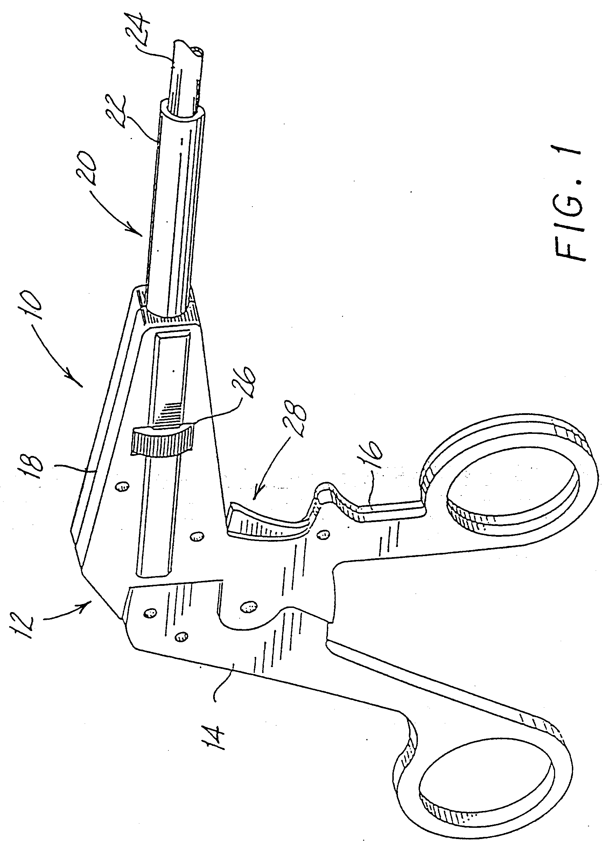

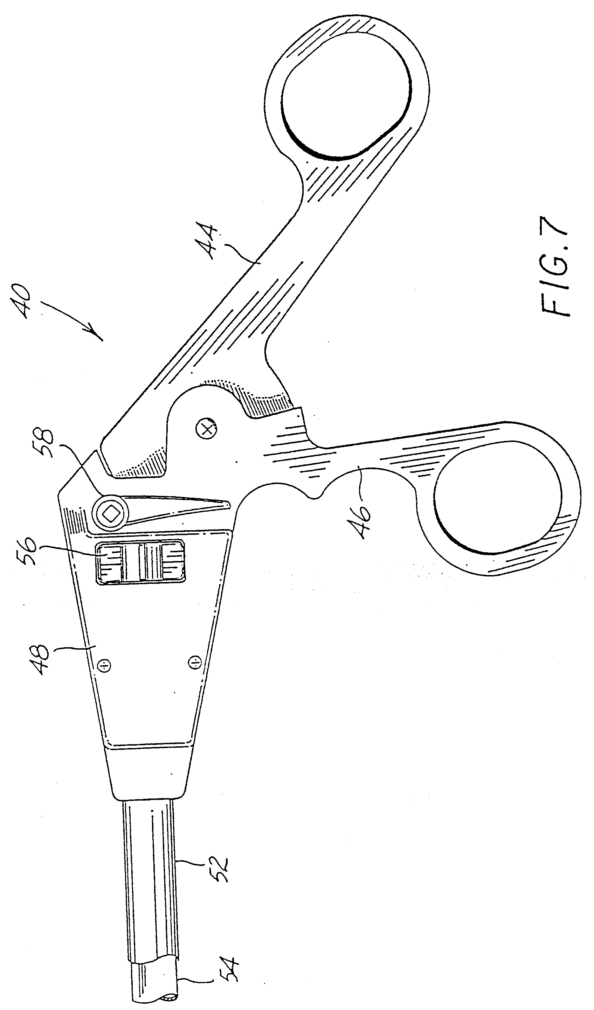

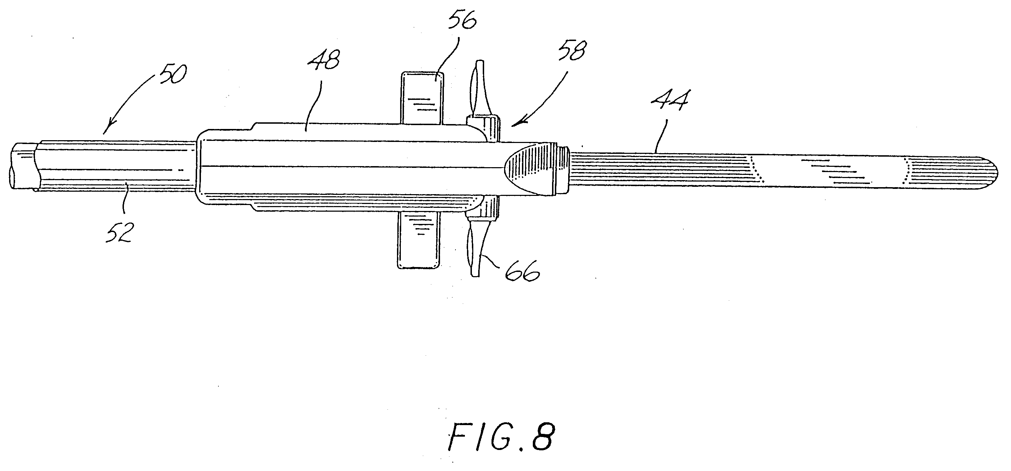

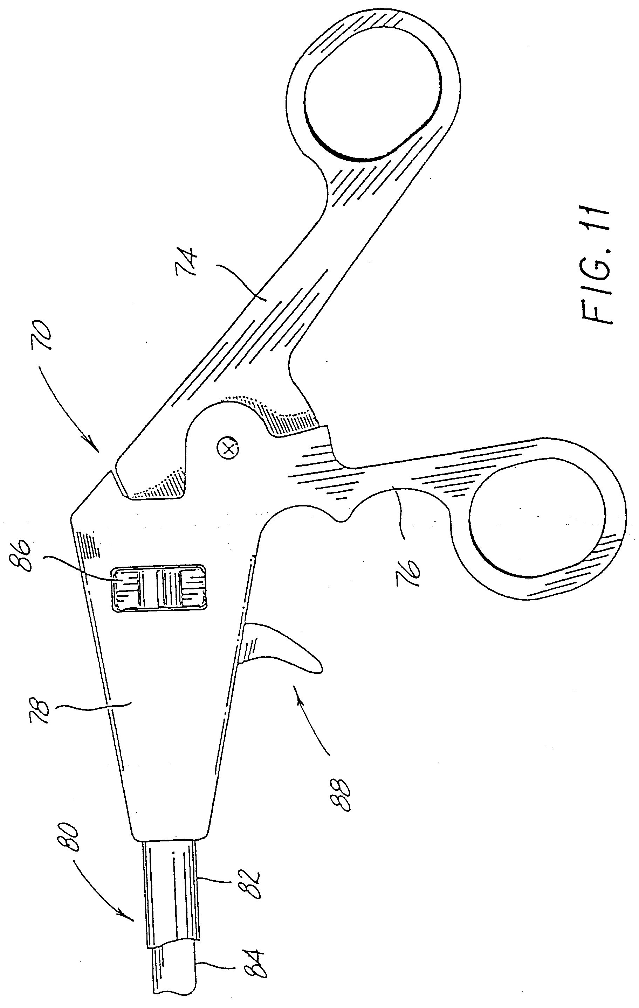

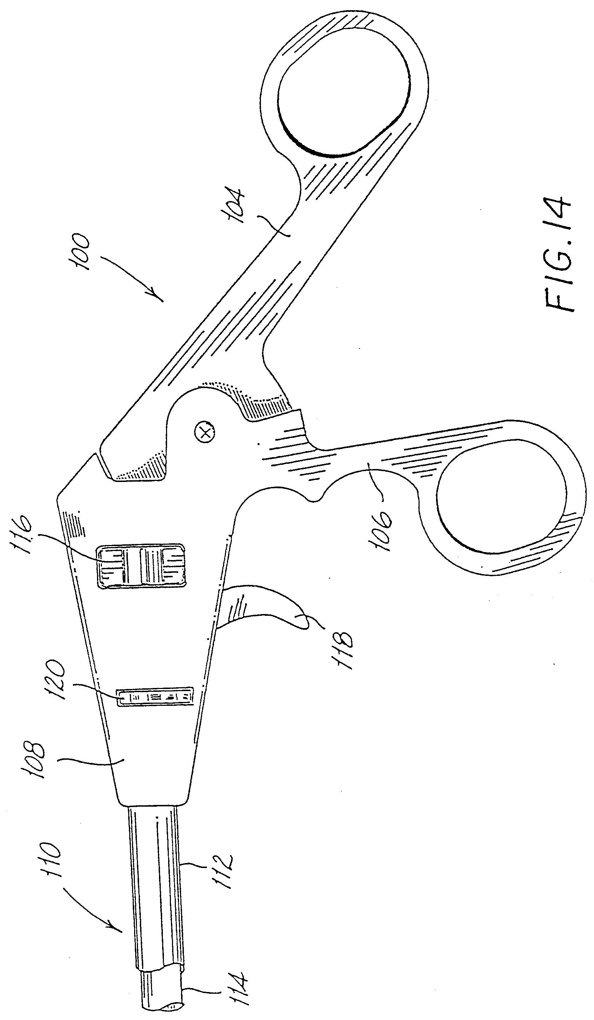

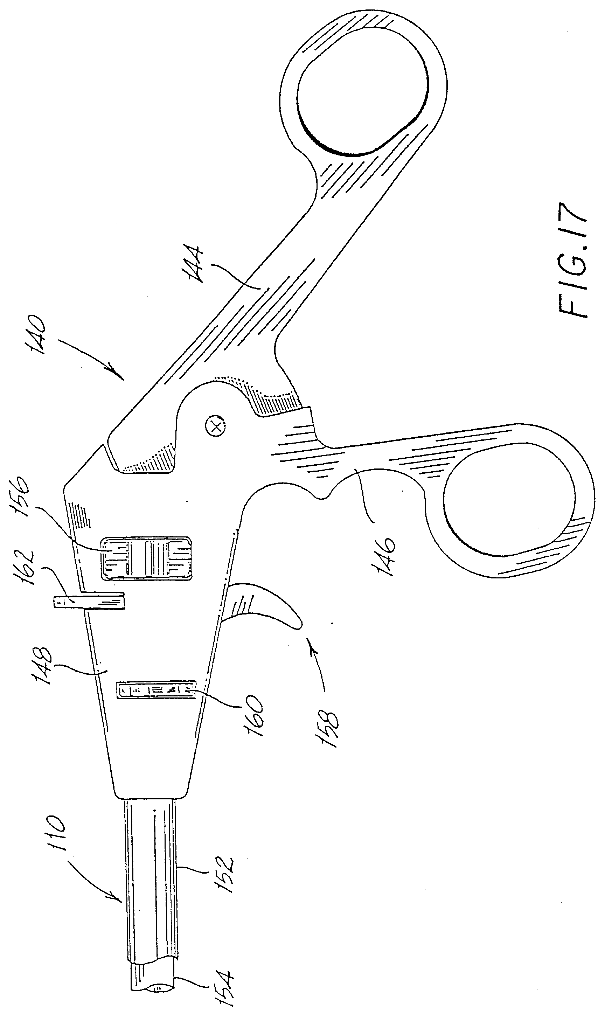

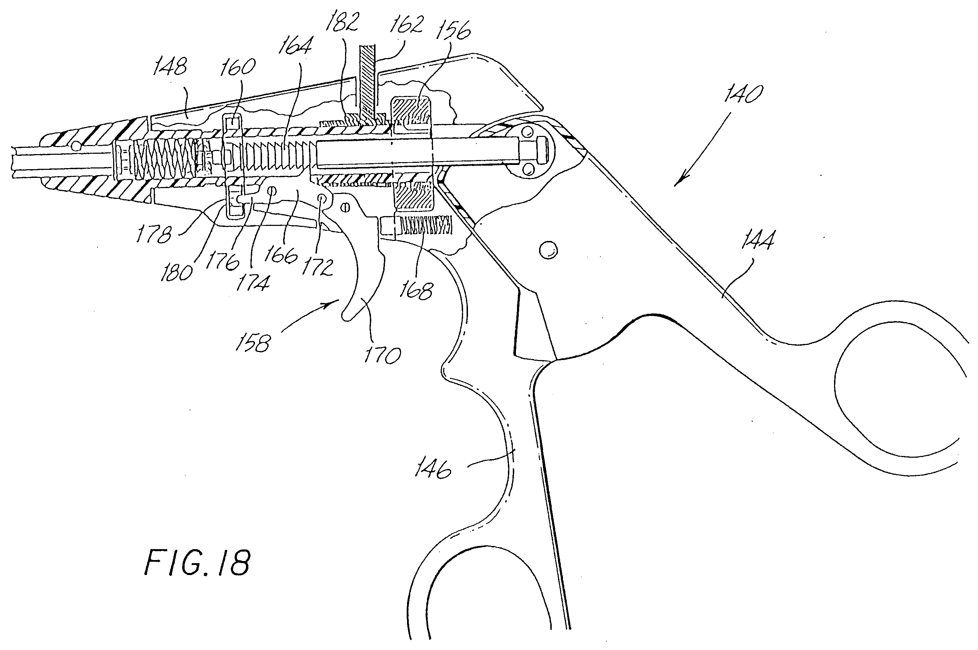

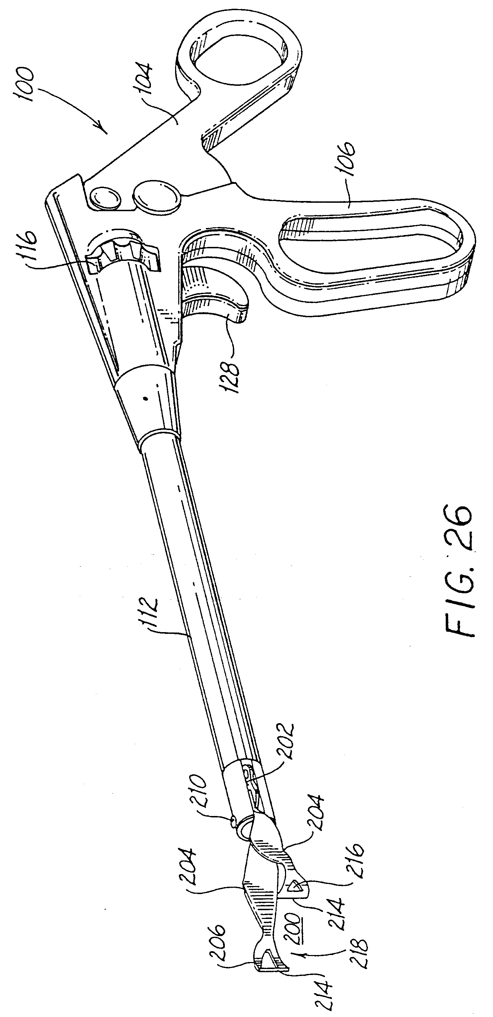

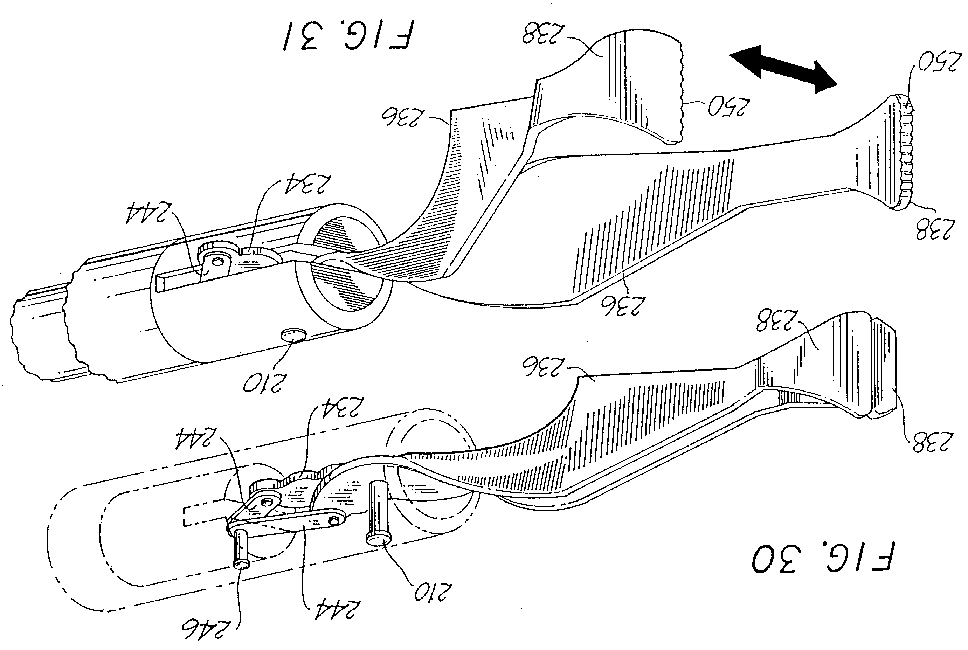

In the drawings, the reference numerals in Figures 1 to 33 are those used in EP-A-0 543 107. Figure 1 illustrates a first embodiment of the endoscopic or laparoscopic surgical instrument 10. In its simplest form, device 10 comprises a handle assembly 12, a body assembly 20, and a ratchet mechanism 28. Handle assembly 12 comprises a pivoting handle 14, a stationary handle 16, and a barrel portion 18 to which body assembly 20 is attached. Body assembly 20 essentially comprises an outer tubular member 22 through which an inner rod member 24 coaxially passes in a slidable arrangement. Preferably, outer tube 22 is secured to barrel portion 18 and remains stationary during operation of the device. Upon movement of pivoting handle 14, inner rod 24 reciprocates within tube member 22 to operate a tool mechanism provided at the distal end of the instrument 10. This tool mechanism (not shown) may comprise a surgical implement, such as scissors, graspers, forceps, retractors and the like. A rotation knob 26 may be provided which rotates body assembly 20 to orient the tool mechanism at various angles to the longitudinal axis.

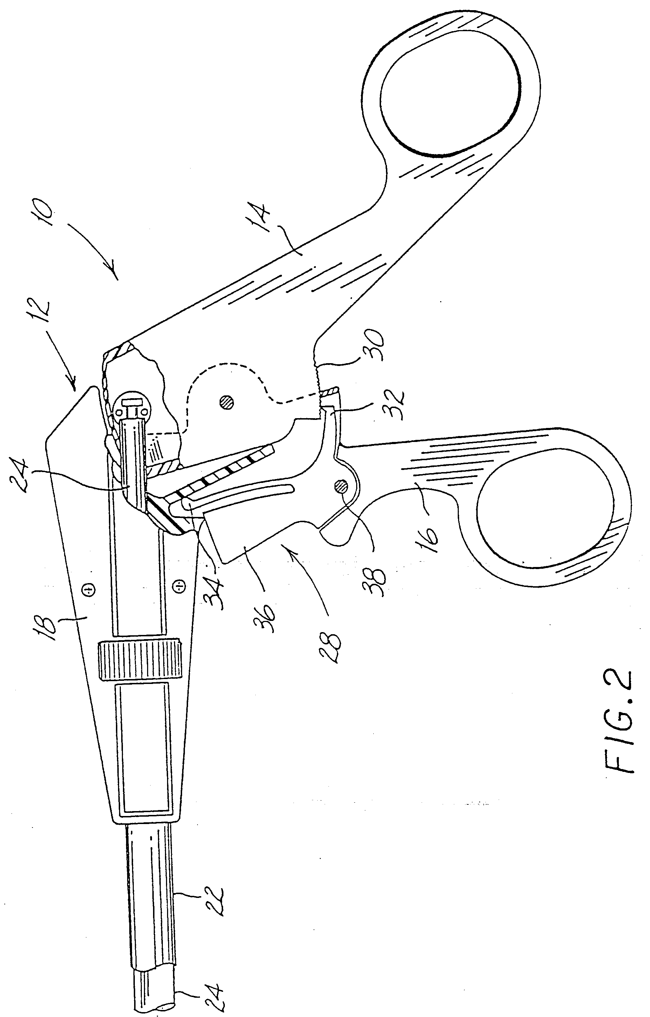

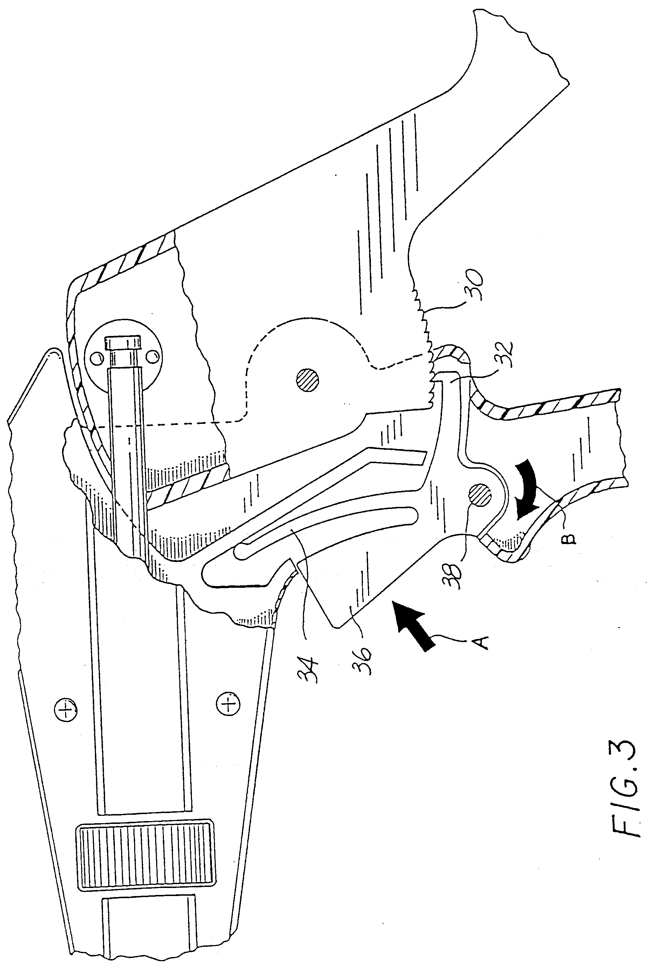

As best seen in Figures 2 and 3, ratchet mechanism 28 is provided to incrementally adjust and hold the position of pivoting handle 14.

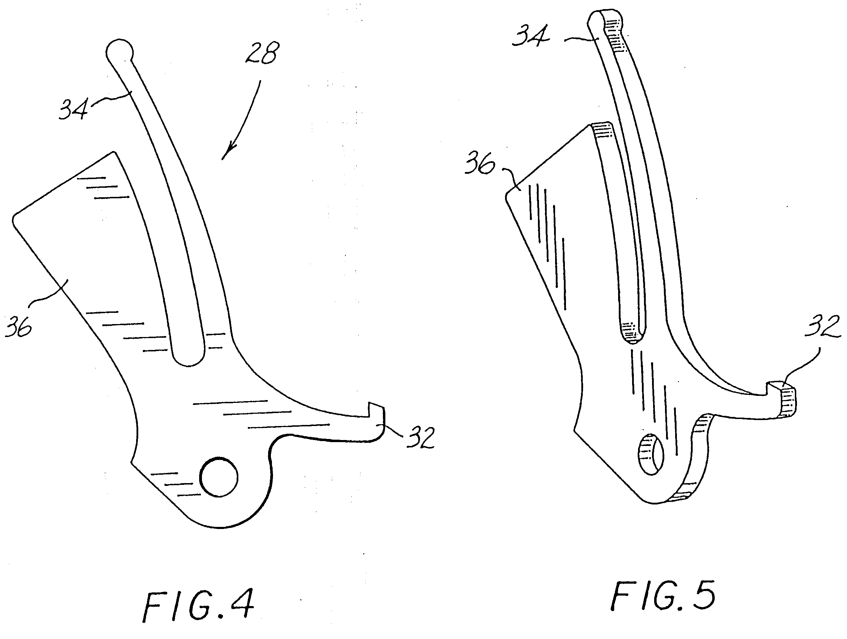

Ratchet mechanism 28 can be best seen in Figures 4 and 5.

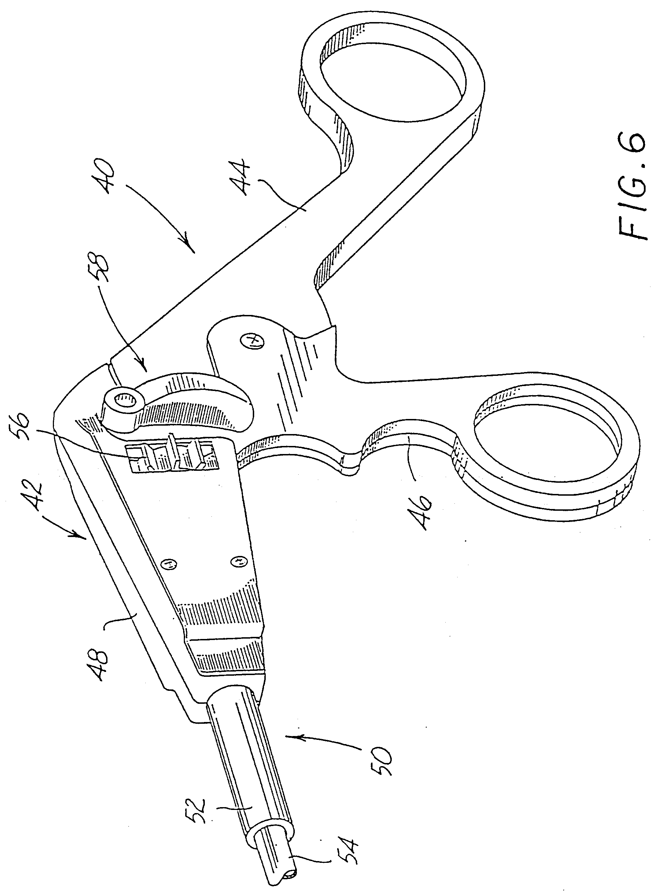

Figure 6 illustrates a second embodiment of the surgical instrument employing the ratchet mechanism of the present invention. Instrument 40 is similar to instrument 10 described above and includes a handle portion 42 to which body assembly 50 is attached. Body assembly 50 terminates in a tool mechanism similar to that described above.

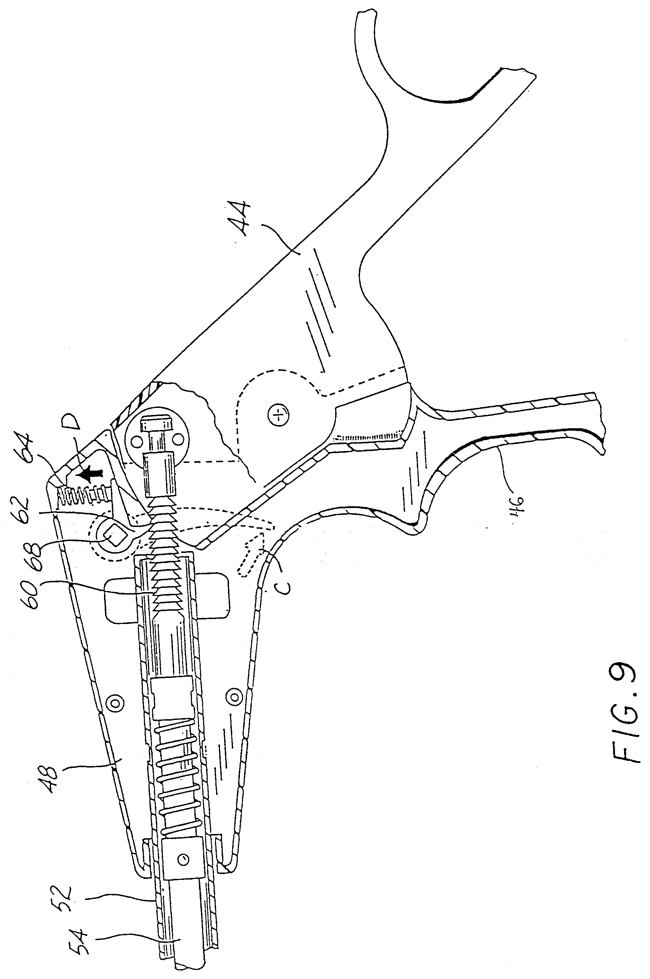

Figure 9 illustrates a cut-away view of the device of Figure 6.

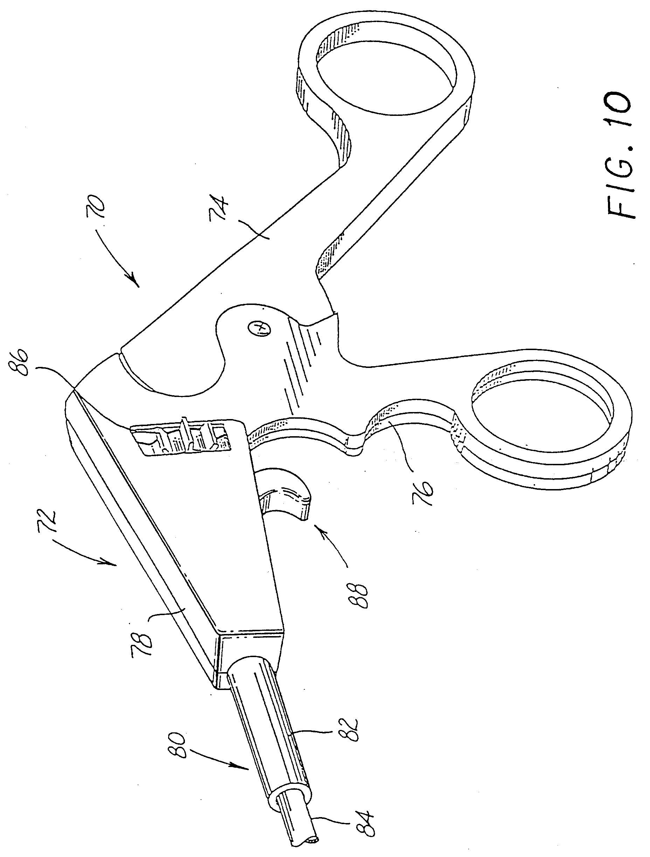

Figure 10 illustrates a third embodiment of the endoscopic or laparoscopic surgical instrument.

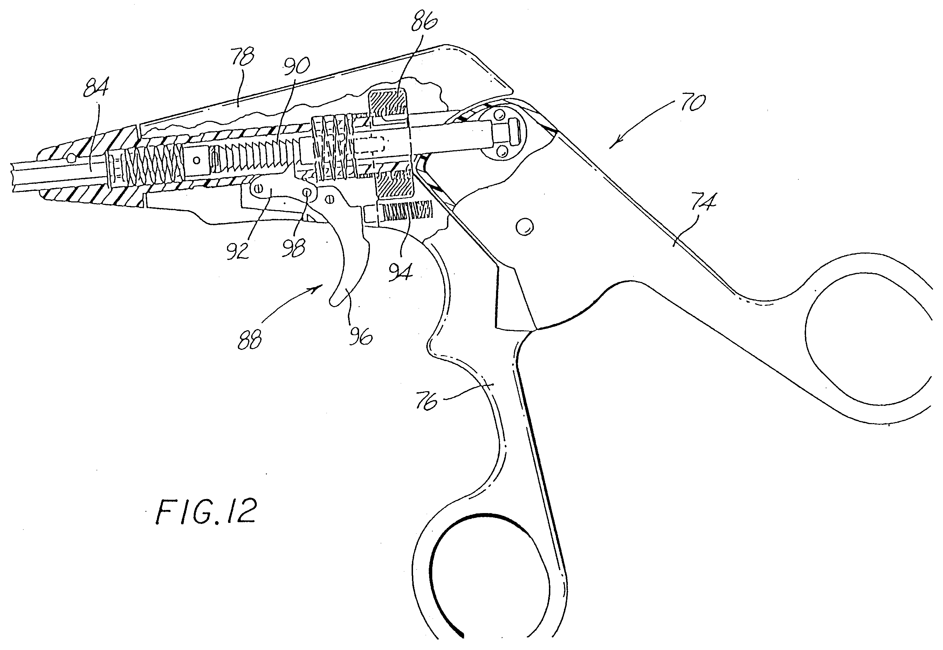

Turning to Figure 12, there is illustrated the ratchet mechanism 88 which is disposed within barrel portion 78.

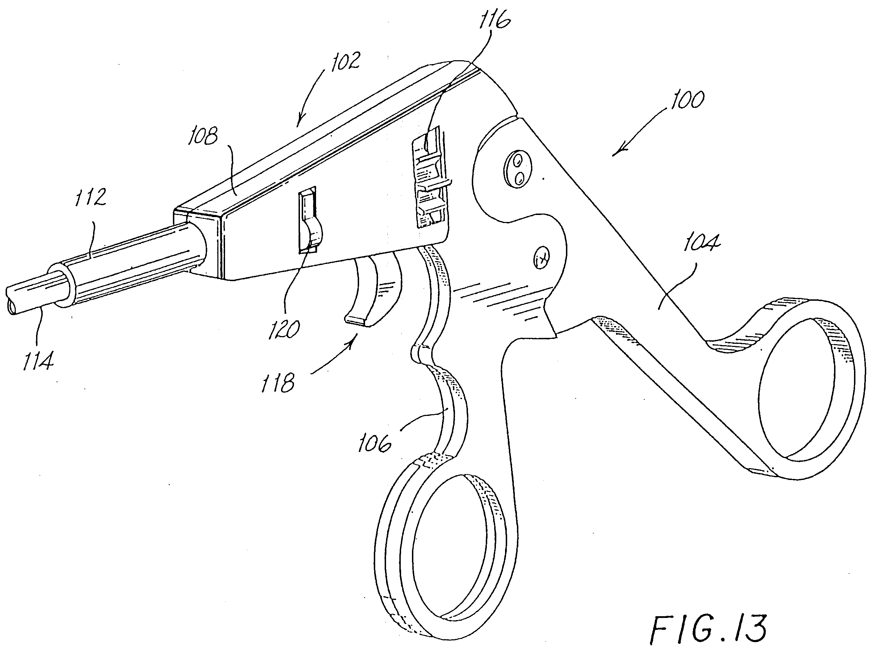

Figure 13 illustrates a fourth embodiment of the endoscopic or laparoscopic surgical instrument 100.

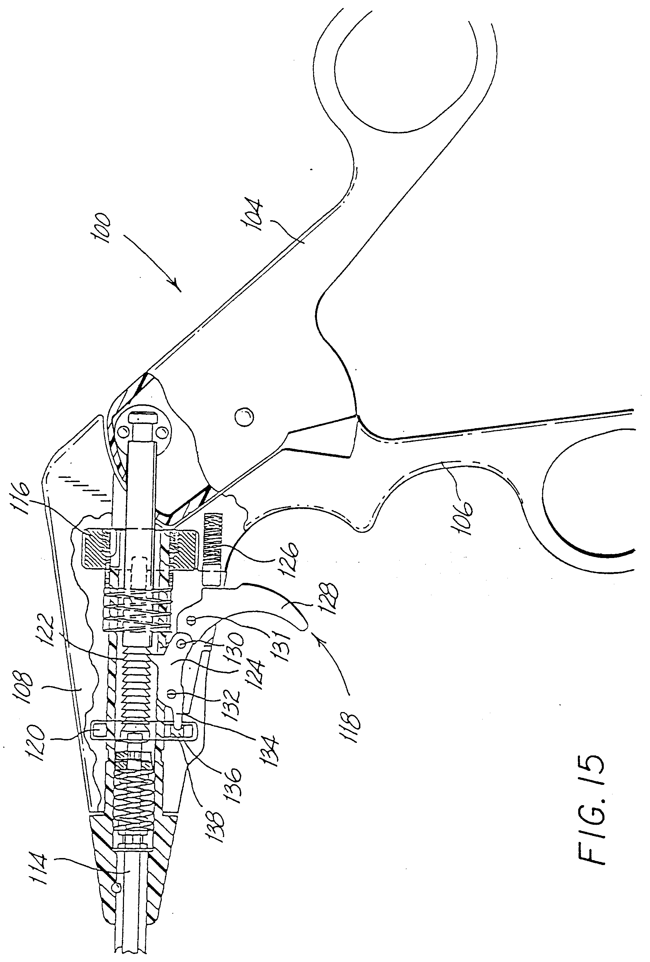

In Figure 15, there is illustrated a side cut-away view of instrument 100 showing the ratchet mechanism 118.

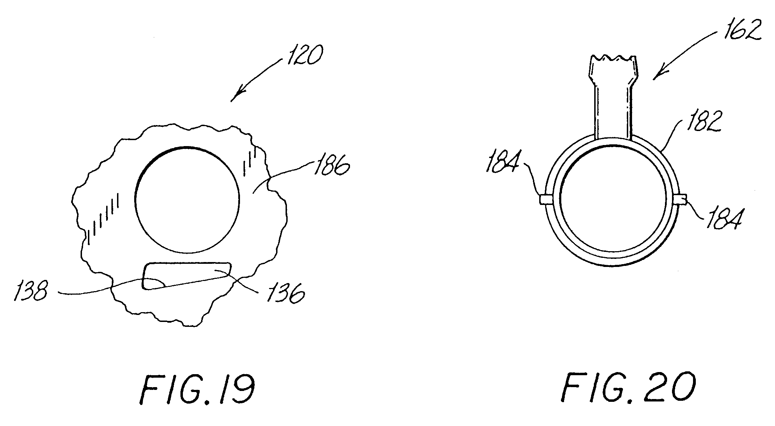

An actuation means 120 is provided, which is best seen in Figure 19.

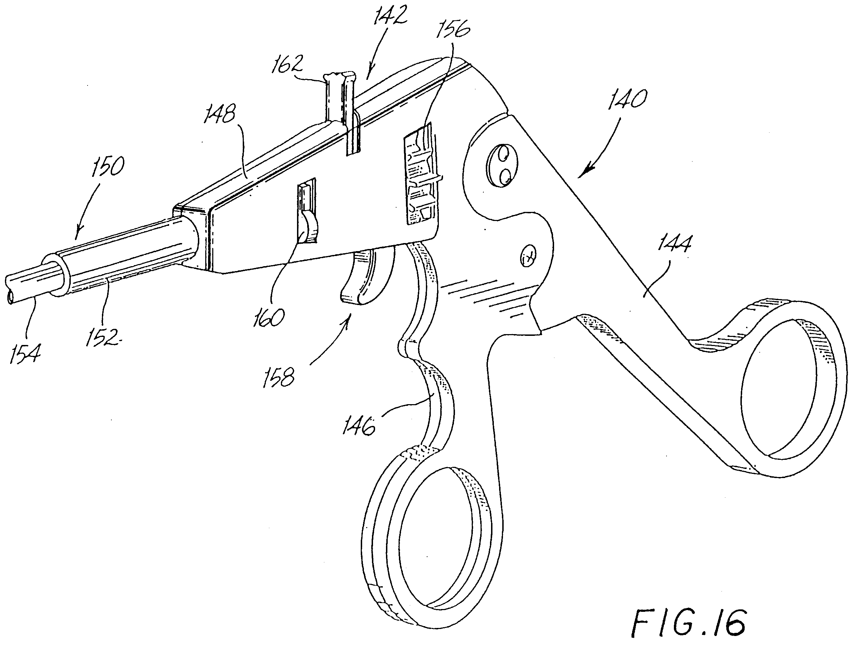

Figure 16 illustrates a fifth embodiment of the endoscopic or laparoscopic surgical instrument 140.

Embodiments of the present invention may further include novel tool mechanisms in the form of atraumatic jaw mechanisms having jaw members with flexural characteristics. These jaw members are capable of transmitting to users a more accurate "feel" so that they can gauge the amount of force being applied to the captured tissue by the instrument. In particularly advantageous embodiments, these jaw members may be fabricated using a unique manufacturing technique which eliminates or substantially reduces the need for elaborate and expensive metal working equipment.

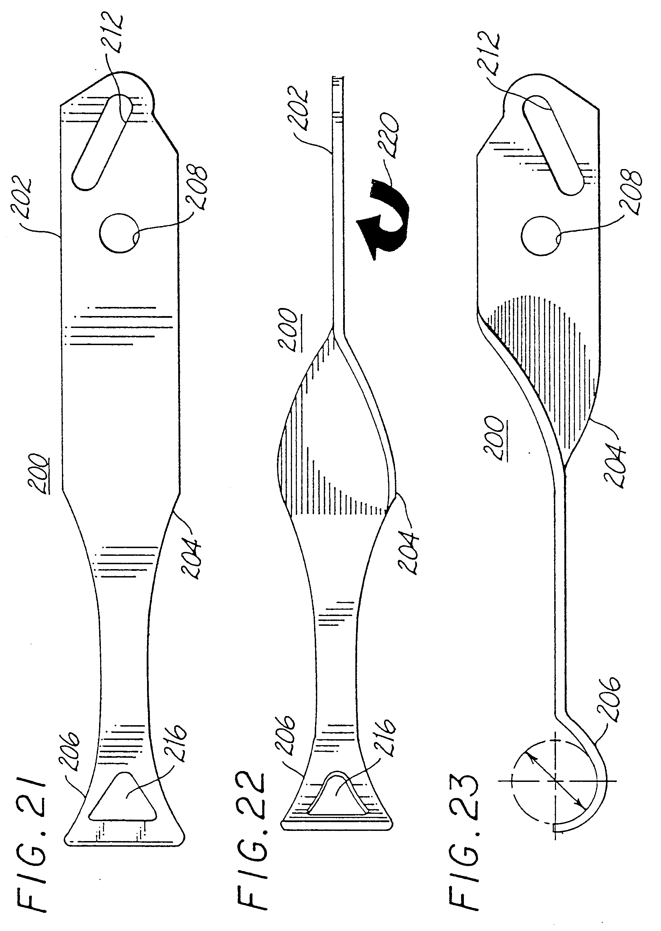

Referring to Figures 21-26 and in particular to Figures 21-23, EP-A-0 543 107 discloses a jaw member and method of manufacturing same is illustrated. This jaw member is shown in the form of a Babcock clamp jaw 200 adapted to capture and hold tubular tissue, preferably without causing traumatic injury. Babcock clamp jaw 200 comprises a proximal portion 202, an intermediate portion 204 and a distal portion 206.

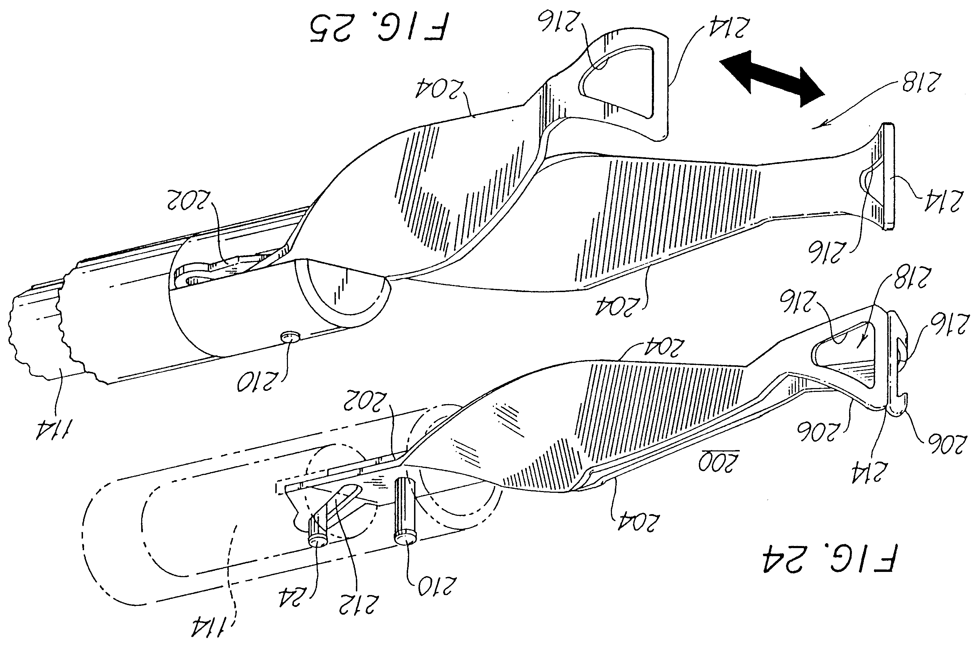

As shown in Figure 21, the proximal portion 202 of the Babcock clamp jaw 200 is provided with a pivot bore 208 transversely formed therein for receiving a pivot pin 210 (Figure 24) to interconnect a pair of jaw members. Proximal portion 202 further includes means for moving the interconnected jaw members pivotally relative to one another. In the embodiment shown in Figures 21-26, this structure comprises a diagonal camming slot 212 formed in the proximal portion 202, which slot receives a camming pin 24 connected to and controlled by inner rod member 114. Note that the jaw structure is described in the context of a surgical instrument in accordance with the embodiment of Figure 15 discussed hereinabove. However, the jaw structure of the present invention may be equally incorporated into other surgical instrument configurations including the alternative embodiments discussed herein.

The distal portion 206 of the Babcock clamp jaw 200 includes a contact surface 214 and a tissue expansion bore 216 formed therein. Tissue expansion bore 216 also provides a fluid passage for fluids associated with grasped tissue. The distal portion is formed into a substantially semicircular shape defining a tissue capturing space 218 on an interior surface thereof.

Intermediate portion 204 is disposed between proximal portion 202 and distal portion 206 and permits the distal portion 206 to flex transversely with respect to proximal portion 202 when the contact surface 214 of two opposing clamp jaw members contact or when an enlarged tissue structure is contained within tissue capturing space 218. In the embodiment of Figures 21-26, the distal portions 206 of clamp jaw member 200 are precambered inward such that as the opposing claim jaws are approximated, the contact surface 214 of the respective distal portions 206 come into abutment first. This configuration allows the intermediate portion 204 to flex or bend thus transmitting a feel to the user of the force being applied.

The Babcock clamp jaws 200 may advantageously be formed in a novel manner from a single sheet of material. Preferably, this material is relatively strong and malleable with good flexural characteristics, e.g. Series 302 or 304 stainless steel.

To form the jaws, the basic shape of the jaw is stamped or formed out of a single flat sheet of material. Thereafter, any desired bores or slots may be formed as desired. Once the basic flat shape is present, the distal end of the flattened workpiece is twisted or bent so that the distal end is disposed in a predetermined angular orientation with respect to the proximal portion of the workpiece. In the embodiment of Figures 21-26, the distal portion 206 is twisted in the direction of arrow 220 (Figure 22) until the distal portion 206 is disposed in a plane approximately 90° transverse to the plane of the proximal portion 202.

Once the workpiece is bent into the correct shape, the distal portion 206 of the jaw member 200 is formed into its final semicircular shape using, for example, a dowel or other forming tool of a predetermined shape. See Figure 23. The distal portion may also be precambered to provide a larger range of flexibility for the jaw members.

The embodiment of Figures 21-26 demonstrate jaw members wherein the distal portions thereof are each bent in the same transverse direction relative to the respective proximal portions.

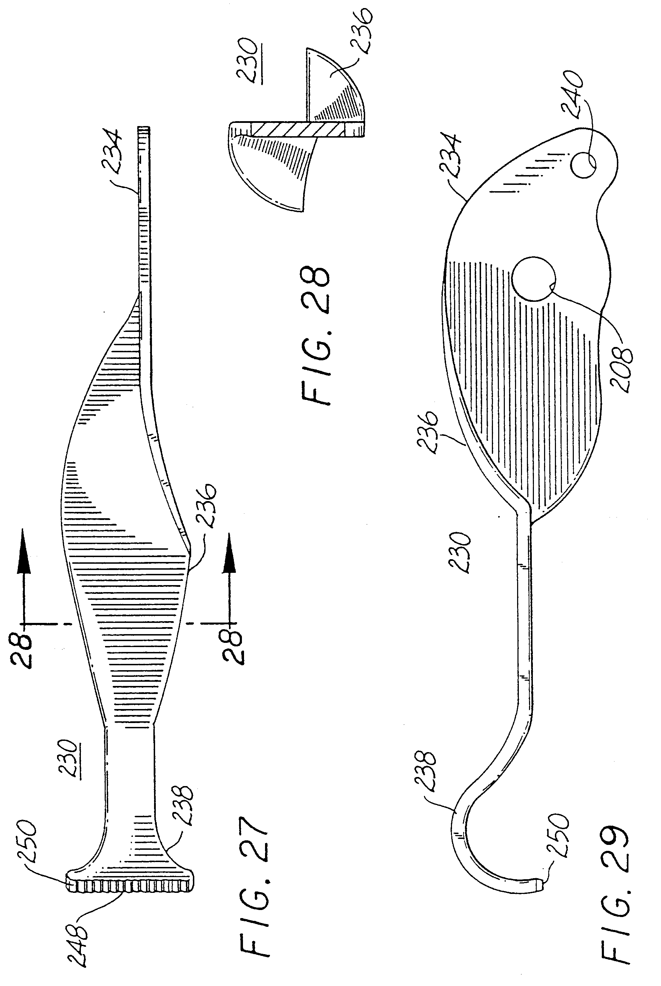

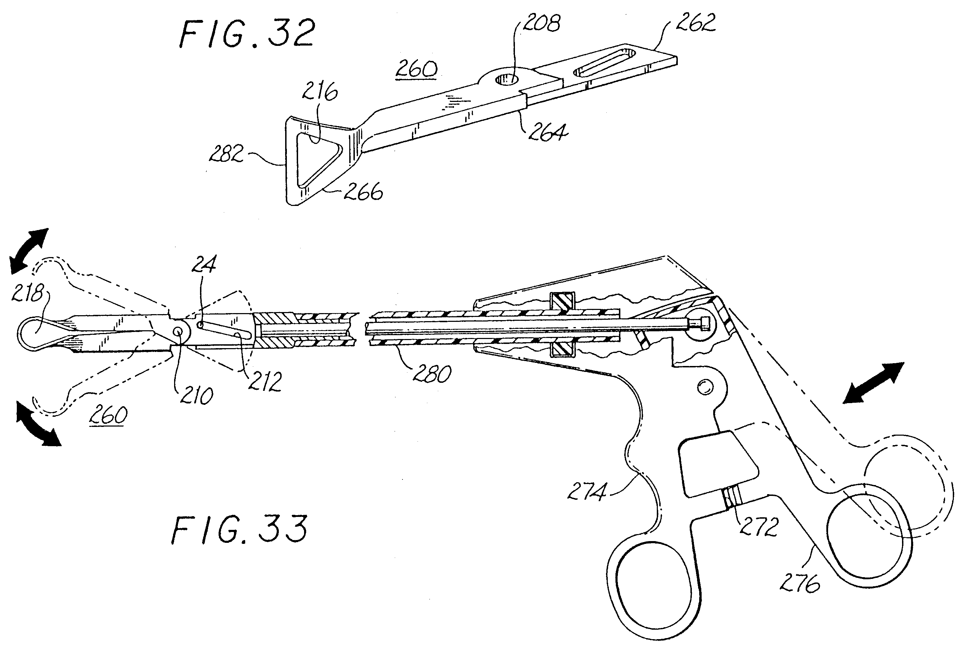

Figures 27-31 illustrate a further embodiment of Babcock clamp jaws 230 and, in Figures 32 and 33, there is illustrated a third configuration of the Babcock clamp jaw.

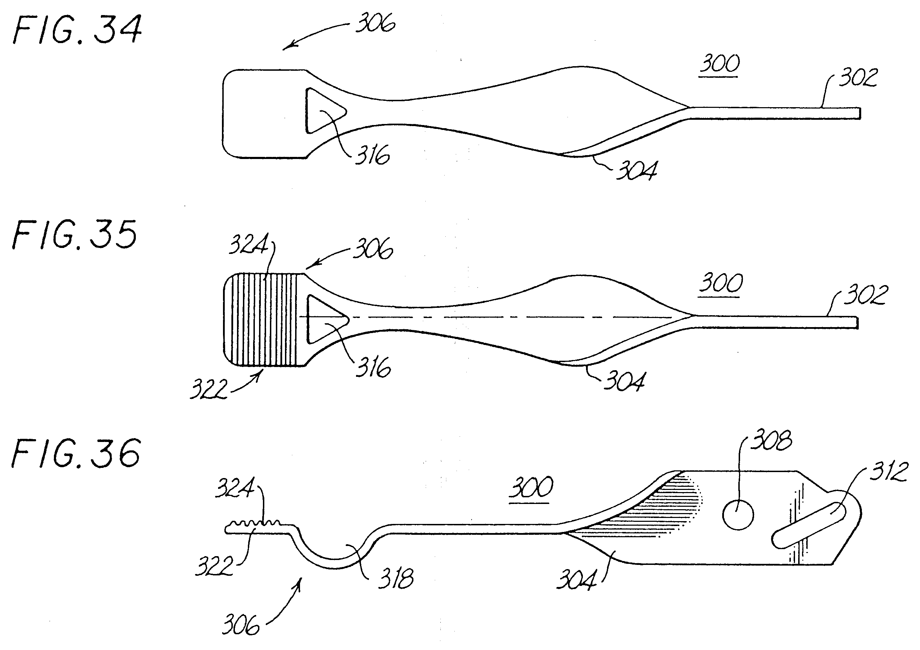

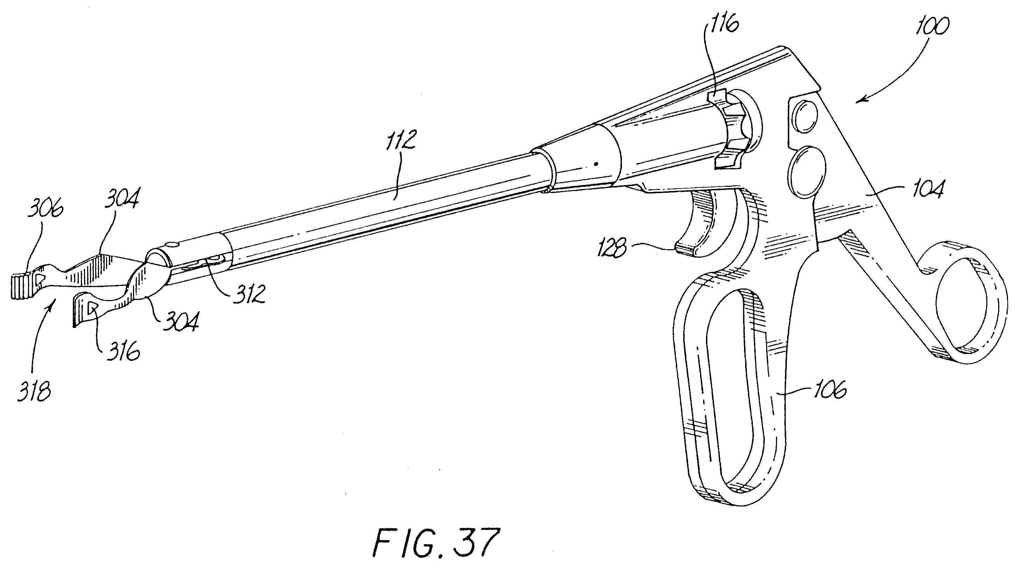

Referring to Figures 34-38 and in particular to Figures 34-36, a jaw member embodiment of the present invention is illustrated. The jaw member is shown in the form of a Babcock clamp and tissue gripper jaw 300 adapted to optionally capture and hold tubular tissue, preferably without causing traumatic injury, or firmly grip tissue. Babcock claim and tissue gripper 300 commprises a proximal portion 302, an intermediate portion 304 and a distal portion 306. Proximal portion 302 and intermediate portion 304 are similar to the corresponding elements as decribed for Figures 21-23 above and will not be discussed further herein.

Distal portion 306 of Babcock clamp and tissue gripper jaw 300 includes tissue expansion bore 316 formed therein. Tissue expansion bore 316 also provides a fluid passage for fluids associated with tissue grasped in tissue capturing space 318 on an interior surface of distal portion 306. Tissue gripping face 322 extends distally away from tissue capturing space 318 and has teeth 324 disposed transversely along tissue gripping face 322. Alternatively, longitudinally extending teeth could be provided.

The embodiment of Figures 34-37 illustrates jaw members wherein the distal portions thereof are each bent in the same transverse direction relative to the respective proximal portions. This advantageous configuration permits the jaws, when assembled together to overlap and minimize visual obstruction of the tissue capturing area 318. The operation of Babcock clamp and tissue gripper jaw 300 is similar to that described above for Figures 21-26, however, the addition of novel tissue gripping face 322 provides additional gripping surface and the further capability of jaw 300 to grasp tissue between tissue gripping faces 322 on opposing jaw members without requiring a separate instrument. This feature provides surgeons with additional gripping surface on the same instrument and allows them to perform multiple functions with the same instrument thereby eliminating the need for additional trocars and access points for additional instruments to the surgical site. Excess instrumentation is, therefore, avoided and the operating area is not unnecessarily cluttered with instrumentation and the associated packaging.

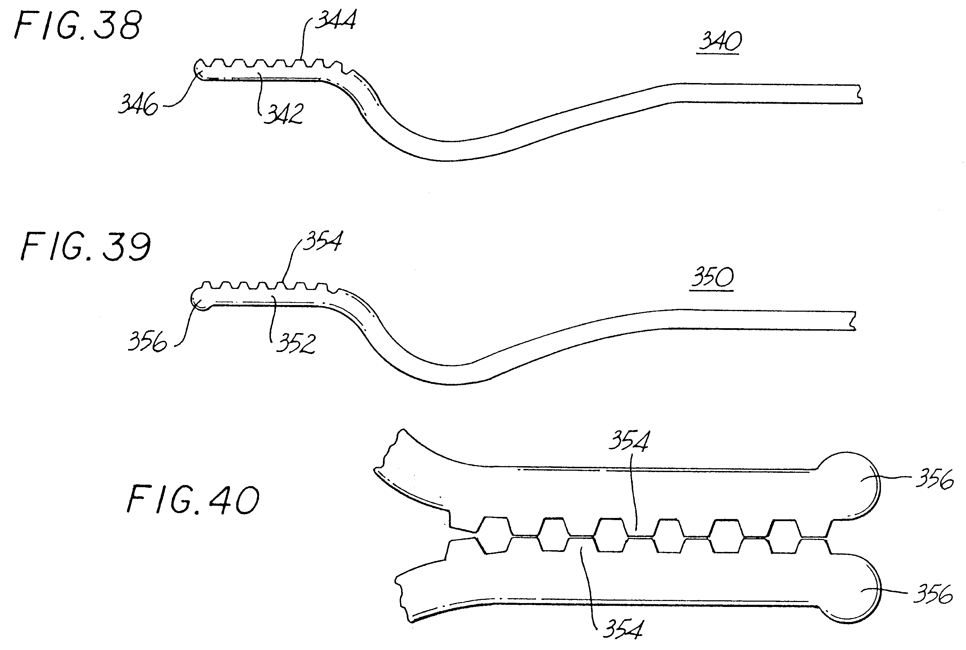

Referring to Figures 38 and 39, Babcock clamp and tissue gripper jaws 340 and 350 are shown featuring alternative tissue gripping faces 342 and 352 having teeth 344 and 354, respectively disposed thereon. Babcock clamp and tissue gripper jaw 340 is rounded at distalmost end 346 while Babcock clamp and tissue gripper jaw 350 has bulbous portion 356 extending downwardly away from teeth 354 for preventing extraneous tissue from being gripped between opposing tissue gripping portions 352. Bulbous portion 356 also reduces the risk of traumatic injury to the surrounding tissue during manipulation of the instrument.

Figure 40 shows one possible alignment of the jaw embodiment of Fig. 39 having opposing jaw members 350 biased so that teeth 354 abut to provide concentrated gripping forces at the planar contact points of abutting teeth 354. Alternatively, jaw members 350 or any of the other jaw embodiments disclosed herein could be configured such that teeth 354 mesh such that tissue gripped between opposing jaw members 350 would be compressed between the teeth and the corresponding gaps therebetween. In the embodiment illustrated in Figure 40, teeth 354 have a truncated semicircular geometry with a flat top contact surface to maximize abutment surface area with the corresponding opposing teeth 354. Alternatively, teeth 354 may be involute as in the geometry of a gear tooth. It has been found that teeth having a total height, i.e., from the top flat contact surface to the bottom of the space between teeth, of 0.254 mm (0.010 inches) a center to center distance between teeth of 0.813 mm (0.032 inches) and a center of tooth to center of the space between teeth distance of 0.406 mm (0.016 inches) is particularly suitable for gripping tissue effectively between opposing jaw members for most purposes. A radius of 0.216 mm (0.0085 inches) for the theoretical semicircle of the truncated semicircular teeth along with a height of 0.203 mm (0.008 inches) from the center of the theoretical circle having the same radius as the semicircle defining the teeth to the flat contact surface has been found particularly suitable. For such a jaw member, bulbous portion 356 having a radius of 0.457 mm (0.018 inches) for its rounded, distally extending shape has been found effective for preventing trauma to tissue both during manipulation of the instrument and during gripping procedures.

While the above embodiments have been described in the context of a Babcock clamp and tissue gripper jaw, other jaw structures may be advantageously formed in this fashion and in these configurations. Examples of other appropriate jaw structures includes graspers, dissectors, forceps, etc.

The claims which follow define embodiments of the invention additional to those described in detail above.

## Claims

1. An endoscopic or laproscopic surgical instrument comprising:
a handle portion;
an endoscopic portion having a proximal end connected to said handle portion and a distal end mounting an atraumatic jaw mechanism, said atraumatic jaw mechanism including a pair of jaw members disposed in opposing relation wherein at least one jaw member is movable such that said jaw members may be positioned in at least an open position and a closed position, each said jaw member having a distal portion (306) and a proximal portion (302), the distal portion defining a capturing space on an interior surface, for atraumatically holding a tubular vessel in the closed position of the jaws, and a gripping area (322);
**characterised in that:**
i. the gripping area comprises a planar face (322);
ii. each jaw member has a flat area within its proximal portion (302) provided with a pivot bore (308) transversely formed therein; and
iii. the gripping face (322) is perpendicular to the flat area in the proximal portion (302); and
iv. an intermediate portion (304) is disposed between the proximal portion (302) and the distal portion (306).

2. An instrument as claimed in claim 1, wherein each jaw member includes a Babcock clamp jaw.

3. An endoscopic or laparoscopic surgical instrument according to either of the preceding claims wherein said capturing space is defined by portions of said jaw members each having a section (318), of substantially semicircular shape such that, when said jaw members are in said closed position, said semicircular sections of said distal portions provide a substantially circular opening adapted for enclosing tissue therein.

4. An instrument according to any one of the preceding claims wherein said planar tissue gripping face is substantially square in shape.

5. An instrument according to any one of the preceding claims wherein at least one of said planar gripping faces includes a plurality of teeth (344, 354) disposed thereon such that when said jaw members are in said closed position tissue may be contacted by said teeth and gripped between said opposing jaw members.

6. An instrument according to claim 5 wherein said teeth are disposed on each of said tissue gripping faces such that when said jaw members are in said closed position said teeth of each of said opposing jaw members abuts or meshes with corresponding teeth of said other opposing jaw member.

7. An instrument according to claim 5 or 6 wherein the teeth extend across the planar gripping face in a direction parallel to the pivot axis.

8. An instrument according to any one of claims 5 to 7 wherein each of said teeth has a truncated semicircular geometry.

9. An endoscopic or laparoscopic surgical instrument according to any one of claims 5 to 7 wherein each of said teeth has an involute geometry.

10. An instrument according to any one of the preceding claims, wherein said jaw member has at its distal end a bulbous portion (356) for reducing trauma to surrounding tissue during manipulation of the jaw.

11. An instrument according to any one of the preceding claims wherein the distal portion of the jaw member (300) defines a tissue expansion bore (316).

12. An instrument according to any one of the preceding claims wherein the intermediate portion (304) between the proximal portion (302) and the distal portion (306) is twisted.

13. An instrument as claimed in any one of the preceding claims, wherein a pivot pin (210) extends through the respective pivot bores (308) with the two respective flat areas surrounding the respective pivot bores arranged face to face.

## Patentansprüche

1. Endoskopisches oder laparoskopisches Operationsinstrument, umfasssend:
einen Griffbereich;
einen endoskopischen Bereich mit einem proximalen Ende, das mit dem Griffbereich verbunden ist, und einem distalen Ende, an dem ein atraumatischer Klemmbackenmechanismus angebracht ist, wobei der atraumatische Klemmbackenmechanismus ein Paar von Klemmbacken umfaßt, die in gegenüberliegender Beziehung angeordnet sind, worin zumindest ein Klemmbackenelement so bewegbar ist, daß die Klemmbackenelemente in zumindest einer offenen Position und einer geschlossenen Position angeordnet werden können, wobei jedes der Klemmbackenelemente einen distalen Bereich (306) und einen proximalen Bereich (302) besitzen, und der distale Bereich einen Erfassungsraum auf einer inneren Oberfläche begrenzt, um ein röhrenförmiges Gefäß in der geschlossenen Position der Klemmbacken atraumatisch zu halten, und einen Greifbereich (322);
dadurch **gekennzeichnet,** daß
i. der Greifbereich eine ebene Fläche (322) umfaßt;
ii. jedes Klemmbackenelement einen flachen Bereich innerhalb seines proximalen Bereiches (302) besitzt, der mit einer Schwenkbohrung (308) versehen ist, die quer verlaufend darin gebildet ist; und
iii. die Greiffläche (322) senkrecht zum flachen Bereich in dem proximalen Bereich (302) ist; und
iv. ein Zwischenbereich (304) zwischen dem proximalen Bereich (302) und dem distalen Bereich (306) angeordnet ist.

2. Instrument gemäß Anspruch 1, worin jedes Klemmbackenelement eine Babcock-Klemmbacke umfaßt.

3. Endoskopisches oder laparoskopisches Operationsinstrument gemäß einem oder beiden der vorhergehenden Ansprüche, worin der Erfassungsraum durch Bereiche des Klemmbackenelements begrenzt ist, die alle einen Bereich (318) von im wesentlichen halbkreisförmiger Form besitzen, so daß, wenn die Klemmbackenelemente in der geschlossenen Position sind, die halbkreisförmigen Abschnitte der distalen Bereiche eine im wesentlichen kreisförmige Öffnung vorsehen, die dazu angepaßt ist, Gewebe darin einzuschließen.

4. Instrument gemäß einem der vorhergehenden Ansprüche, worin die ebene Gewebegreiffläche eine im wesentlichen quadratische Form besitzt.

5. Instrument gemäß einem der vorhergehenden Ansprüche, worin zumindest eine der ebenen Greifflächen eine Vielzahl von Zähnen (344, 354) umfaßt, die darauf so angeordnet sind, daß, wenn die Klemmbackenelemente in der geschlossenen Position sind, Gewebe durch die Zähne berührt und zwischen den gegenüberliegenden Klemmbackenelementen gegriffen werden kann.

6. Instrument gemäß Anspruch 5, worin die Zähne auf jeder der Gewebegreifflächen so angeordnet sind, daß, wenn die Klemmbackenelemente in der geschlossenen Position sind, die Zähne jeder der gegenüberliegenden Klemmbackenelemente mit entsprechenden Zähnen des anderen gegenüberliegenden Klemmbackenelements anstoßen oder mit diesen kämmen.

7. Instrument gemäß Anspruch 5 oder 6, worin sich die Zähne über die ebene Greiffläche in einer Richtung parallel zur Schwenkachse erstrecken.

8. Instrument gemäß einem der Ansprüche 5 bis 7, worin jeder der Zähne eine abgeschnittene halbkreisförmige Geometrie besitzt.

9. Endoskopisches oder laparoskopisches Operationsinstrument gemäß einem der Ansprüche 5 bis 7, worin jeder der Zähne eine evolventenförmige Geometrie besitzt.

10. Instrument gemäß einem der vorhergehenden Ansprüche, worin das Klemmbackenelement an seinem distalen Ende einen kugelförmigen Bereich (356) besitzt, um eine Verletzung des umgebenden Gewebes während der Betätigung der Klemmbacke zu verringern.

11. Instrument gemäß einem der vorhergehenden Ansprüche, worin der distale Bereich des Klemmbackenelements (300) ein Gewebeausbreitungsloch (316) begrenzt.

12. Instrument gemäß einem der vorhergehenden Ansprüche, worin der Zwischenbereich (304) zwischen dem proximalen Bereich (302) und dem distalen Bereich (306) verwunden ist.

13. Instrument gemäß einem der vorhergehenden Ansprüche, worin ein Schwenkstift (210) sich durch die jeweiligen Schwenkbohrungen (308) erstreckt, wobei die zwei jeweiligen flachen Bereiche, welche die jeweiligen Schwenkbohrungen umgeben, aufeinander zu gerichtet angeordnet sind.

## Revendications

1. Instrument chirurgical endoscopique ou laparoscopique comportant :
une partie formant poignée;
une partie endoscopique comportant une extrémité proximale reliée à ladite partie de poignée et une extrémité distale sur laquelle est montée un mécanisme à mâchoires ne provoquant pas de trauma, ledit mécanisme à mâchoires ne provoquant pas de trauma incluant une paire d'éléments de mâchoire disposée suivant une relation opposée, dans lequel au moins un élément de mâchoire est déplaçable de façon que lesdits éléments de mâchoire peuvent être positionnés suivant au moins une position ouverte et une position fermée, chacun desdits éléments de mâchoire comportant une partie distale (306) et une partie proximale (302), la partie distale définissant un espace de saisie sur une surface intérieure, pour retenir, sans provoquer de trauma, un vaisseau tubulaire dans la position fermée des mâchoires, et une zone de prise (322);
**caractérisé en ce que :**
i. la zone de prise comporte une face plane (322);
ii. chaque élément de mâchoire comporte une zone plate dans sa partie proximale (302) pourvue d'un perçage de pivot (308) ménagé transversalement dans celle-ci; et
iii. la face de saisie (322) est perpendiculaire à la zone plate dans la partie proximale (302); et
iv. une partie intermédiaire (304) est disposée entre la partie proximale (302) et la partie distale (306).

2. Instrument selon la revendication 1, où chaque élément de mâchoire comporte une mâchoire de serrage Babcock.

3. Instrument chirurgical endoscopique ou laparoscopique selon l'une des revendications précédentes, dans lequel ledit espace de saisie est défini par des parties desdits éléments de mâchoire chacune présentant une section (318) de forme sensiblement semi-circulaire de manière que lorsque lesdits éléments de mâchoire se trouvent dans ladite position fermée, lesdites sections semi-circulaires desdites parties distales réalisent une ouverture sensiblement circulaire conçue pour renfermer le tissu à l'intérieur.

4. Instrument selon l'une des revendications précédentes, dans lequel ladite face de saisie de tissu plane a une forme sensiblement carrée.

5. Instrument selon l'une des revendications précédentes, dans lequel au moins l'une desdites faces de saisie planes comporte une pluralité de dents (344, 354) disposée sur celle-ci de façon que lorsque lesdits éléments de mâchoire se trouvent dans ladite position fermée, lesdites dents peuvent venir en contact avec le tissu, et le tissu sera saisi entre lesdits éléments de mâchoire opposés.

6. Instrument selon la revendication 5, dans lequel lesdites dents sont disposées sur chacune desdites faces de saisie de tissu de façon que lorsque lesdits éléments de mâchoire se trouvent dans ladite position fermée, les dents de chacun desdits éléments de mâchoire opposés viennent en butée ou engrènent avec les dents correspondantes dudit autre élément de mâchoire opposé.

7. Instrument selon la revendication 5 ou 6, dans lequel les dents s'étendent sur la face de saisie plane dans une direction parallèle à l'axe de pivotement.

8. Instrument selon l'une des revendications 5 à 7, dans lequel chacune desdites dents a une géométrie semi-circulaire tronconique.

9. Instrument chirurgical endoscopique ou laparoscopique selon l'une des revendications 5 à 7, dans lequel chacune desdites dents a une géométrie à développante.

10. Instrument selon l'une des revendications précédentes, dans lequel ledit élément de mâchoire comporte à son extrémité distale une partie en bulbe (356) pour diminuer le trauma exercé sur le tissu environnant pendant la manipulation de la mâchoire.

11. Instrument selon l'une des revendications précédentes, dans lequel la partie distale de l'élément de mâchoire (300) définit un perçage d'expansion de tissu (316).

12. Instrument selon l'une des revendications précédentes, dans lequel la partie intermédiaire (304) entre la partie proximale (302) et la partie distale (306) est tordue.

13. Instrument selon l'une des revendications précédentes dans lequel un pivot (210) s'étend à travers les perçages de pivot respectifs (308), les deux zones plates respectives entourant les perçages de pivot respectifs agencés face à face.
